Europäisches Patentamt

European Patent Office

Office européen des brevets

(11) Publication number: **0 165 519**
**B1**

(12) **EUROPEAN PATENT SPECIFICATION**

(45) Date of publication of patent specification: **13.06.90**

(51) Int. Cl.⁵: **A 61 M 1/34**

(21) Application number: **85106708.2**

(22) Date of filing: **31.05.85**

(60) Divisional application **89118782.5 filed on 31/05/85.**

(54) A blood filtering system.

(30) Priority: **18.06.84 SE 8403244**

(43) Date of publication of application:
**27.12.85 Bulletin 85/52**

(45) Publication of the grant of the patent:
**13.06.90 Bulletin 90/24**

(84) Designated Contracting States:
**BE CH DE FR GB IT LI NL**

(56) References cited:
**EP-A-0 062 913**
**EP-A-0 087 171**
**DE-A-2 636 290**
**DE-A-3 111 061**
**DE-A-3 202 582**
**DE-A-3 243 523**
**GB-A-2 009 863**
**US-A-4 198 971**

(73) Proprietor: **Gambro Lundia AB**
**Box 10101**
**S-220 10 Lund (SE)**

(72) Inventor: **Ohlsson, Lars Ingvar**
**Ekebergslundsvägen 8**
**S-292 02 Asarum (SE)**
Inventor: **Simonsson, Tord Benny**
**Stora Norregatan 128**
**S-261-40 Landskrona (SE)**
Inventor: **Dahlberg, Bengt Ake Gustav**
**Dörröd 7**
**S-240 14 Veberöd (SE)**
Inventor: **Jeppsson, Jan-Bertil**
**Västkustvägen 86**
**S-234 00 Lomma (SE)**

(74) Representative: **Boberg, Nils Gunnar Erik**
**Gambro AB Patent Department Box 10101**
**S-220 10 Lund (SE)**

## Description

Technical Field

The present invention relates to a blood filtering system comprising means for the withdrawal of blood from a patient or some other source, a filter by means of which a filtrate can be withdrawn from the blood, a duct with a pump for sucking the filtrate from the filter to a flow meter, as well as suitable means for the return of the remainder of the blood to the source together with any replacement fluid. Such a system is disclosed in for instance Fig. 6 of DE-A-3 111 061.

The invention is intended in particular to be used in conjunction with haemofiltration or plasmafers where a replacement fluid is supplied in place of the filtrate withdrawn. It will be clear, however, to those versed in the art that the invention can also be applied in other connections e.g. in conjunction with haemodialysis when the dialysis is combined with the removal of an ultrafiltrate.

Background Art

Haemofiltration with preparation at the same time of replacement fluid is described, for example, in the European patent applications published under no EP 0 042 939 and EP 0 087 171.

Plasmafers differ from haemofiltration principally in that they require a slightly more permeable membrane material for the filtering out of even larger molecules.

Examples of a suitable haemofiltration membrane are described in the European patent application published under no EP 0 046 816. In the same manner a suitable membrane for plasmafers is described in the European patent application published under no EP 0 044 958. It is clear, however, to those versed in the art that other membranes too can be used in connection with the realization of the present invention.

When it is intended in conjunction with, for example, haemofiltration or plasmafers to measure the amounts of filtrate withdrawn it has been found difficult to carry out exact measurements owing to the varying pressure conditions and the consequently varying conditions of flow. Special problems were encountered on application of the peristaltic pumps normally used. These pumps act from the outside upon the flexible blood tubes in a pulsating manner.

In accordance with a preferred embodiment of the present invention a pressure equalizing device comprising a gas-permeable but liquid-tight membrane is used. Reference is made in this connection to the US patent 4 198 971 which describes a drip chamber of a similar design. See also EP-A-0 062 913 which describes another similar drip chamber which, however, has the drawback that the membrane may be blocked, if a condensate is created above the membrane.

Disclosure of Invention

The abovementioned problem is solved in accordance with the invention by means of a blood filtering system of the aforementioned type characterized by a pressure equalizing device arranged in the duct between the pump and the flow meter for the equalizing of the pressure in the flow of filtrate conducted through the measuring device.

This pressure equalizing device is arranged preferably in close connection to the measuring device. In this way it is ensured additionally that the pressure in the measuring device, and consequently also the flow through the same, can be kept substantially constant so that in spite of all variations of flow which occur continuous measurement is readily possible.

The pressure equalizing device is appropriately adapted also to capture any gas bubbles occuring in the flow of filtrate, since these too may disturb the measurement in a subsequent flow meter.

If a peristaltic pump is used in a manner known in itself to act from the outside upon the filtrate conducted in a flexible tube, a contamination of the same is avoided.

It is appropriate to arrange a measuring device also for the amount of replacement fluid supplied. A common measuring device may be used possibly for the measurement of the filtrate as well as of the replacement fluid. This is suitably adapted to compare the fluid flows measured. In this way it can be safely checked that the amounts of fluid withdrawn and supplied are in the specified proportional relationship with each other.

The said second pressure equalizing arrangement can be arranged in a duct for the supply of replacement fluid to the said source, in particular a patient, and is located appropriately behind the measuring device for this fluid.

Preferably at least one of the said pressure equalizing devices is provided with a suitable automatic level control. This can be achieved in a simple manner in that the device is provided with a window to the outer atmosphere covered by a membrane which is gas-permeable but liquid-tight.

The said window may be arranged on the outer wall of a pressure sleeve enclosing the pressure equalizing device with the membrane arranged in vertical position, so that the level normally will vary between the highest and lowest points of the membrane. Alternatively the said window can be arranged inside a housing enclosing the pressure equalizing device, one side of the membrane being adapted to be in contact with the interior of the housing and its other side with the outer atmosphere.

Preferably at least one of the said pressure equalizing devices, and appropriately the firstmentioned one, is provided with means for the prevention of frothing or for the breaking down of froth already formed.

As a further safeguard of the measurements in the said measuring device means may be provided for the measuring of the temperature of the medium flowing through the same. In this manner the measuring result can be compensated on account of the change of density which is obtained at a variation in temperature. By way of an example it may be mentioned that a temperature variation of approx. 2°C gives a change of density in plasma of approx. 1 per mil.

Brief Description of Drawings

Fig. 1 shows in the form of a block diagram a preferred, substantially complete, system in accordance with the invention.

Fig. 2, 3, 4 and 5 show alternative preferred embodiments of pressure equalizing devices included in the system in accordance with the invention.

Best mode of carrying out the Invention

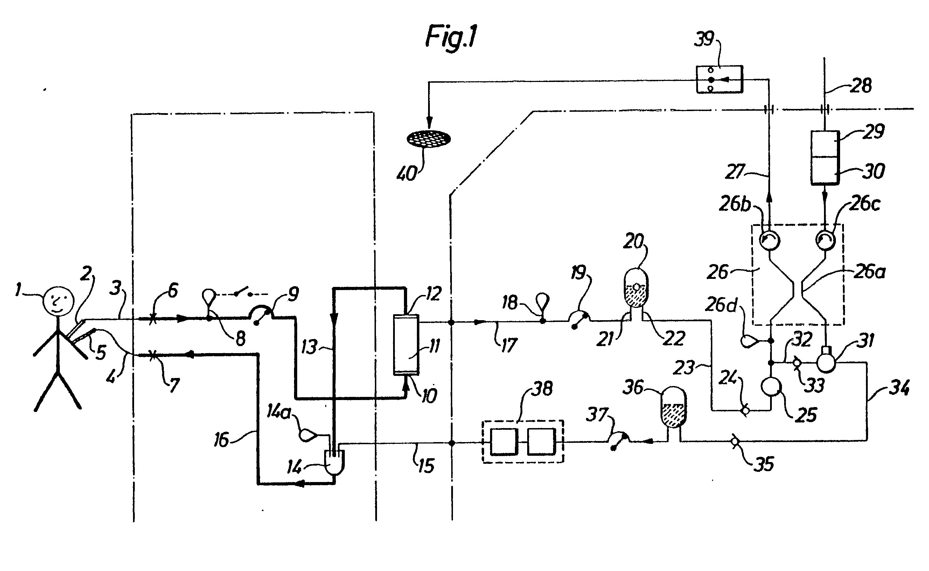

In the block diagram shown in Fig. 1 a patient is designated 1. Blood is withdrawn from him by means of a cannula 2 and is conducted via a duct 3 into a blood treatment system. The blood is returned thereafter to the patient via a duct 4 and a cannula 5. For reasons of safety the ducts can be shut off and opened respectively with the help of clamps 6 and 7. After the clamp 6 the blood passes an arterial pressure gauge 8 arranged upstream of a peristaltic pump 9 by means of which the blood circulation is brought about. From the pump 9 the blood is pressed via an inlet 10 into a filter 11 and out from this via an outlet 12.

The invention is intended to be applied in particular in conjunction with haemofiltration or plasmafers and the filter thus consists here of a haemofilter or a plasmaferfilter respectively.

From the outlet 12 the blood is taken via a duct 13 to a drip chamber 14 which is coordinated with a venous pressure gauge 14a. A replacement fluid is also conducted to the drip chamber 14 via a duct 15. From the drip chamber the blood mixed with replacement fluid is then conducted via a duct 16, the clamp 7 and the cannula 5 back to the patient.

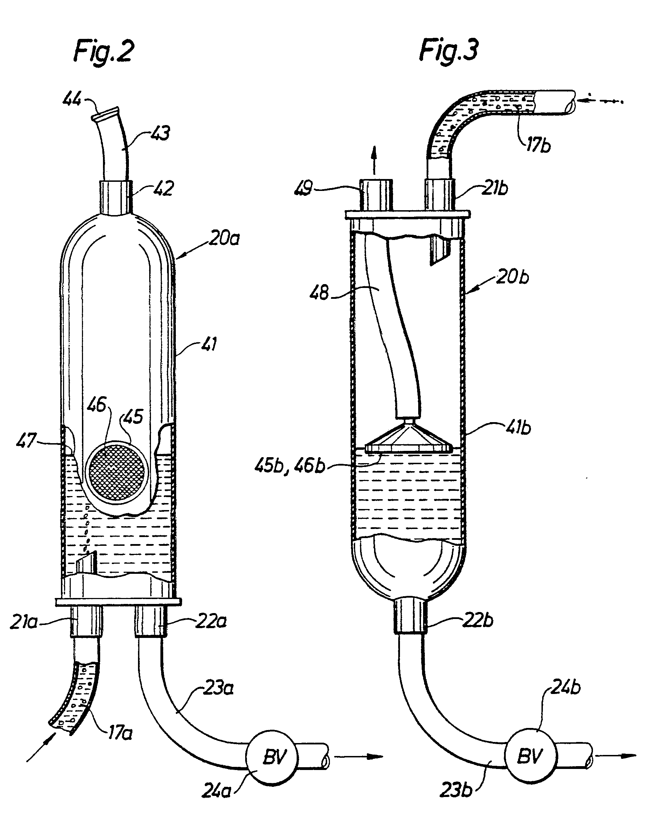

From the filter 11 filtrate is withdrawn via a duct 17, the pressure being measured by means of a pressure gauge 18. To suck the filtrate from the filter 11 a pump 19 is used which preferably is constituted in conventional manner of a peristaltic pump. The filtrate is then introduced into a pressure equalizing device 20 which will be described in more detail in connection with Fig. 2, 3, 4 and 5. The inlet to the pressure equalizing device is designated 21 and the outlet 22. Subsequently the filtrate is conducted by means of a duct 23 via a check valve 24 and an appropriate controllable ultrafiltration valve 25 to a flow meter 26. This may be designed, for example, in accordance with the European patent application, which has been published under no. EP 0 106 940, but can be substituted, of course, by other designs.

From the meter 26 the filtrate is taken via a duct 27 either to a collecting point, if it is to be retained, or to a drain 40.

A replacement fluid prepared in a separate setup is supplied to the meter 26 via a duct 28, a conductivity meter 29 and a temperature measuring device 30.

The meter 26 appropriately comprises a first part 26a with the help of which the difference between flows in the ducts 27 and 28 is measured. As a check, moreover, a measurement of the individual flows in parts 26b and 26c may also take place. For a possible adjustment of the measuring result in relation to the temperature the meter 26 may be coordinated, moreover, with a temperature measuring device 26d. From the meter 26 the replacement fluid is taken to a valve 31 which as a function of the values measured on the measuring devices 29 and 30 conducts replacement fluid either to a by-pass duct 32 via a check valve 33 and from there further to the discharge pipe 27 or to a duct 34 and via a check valve 35, a pressure equalizing device 36, an infusion pump 37 and possibly a filter system 38 and the duct 15 to the drip chamber 14.

Numeral 39 in Fig. 1 finally designates a valve which allows flow only when pressure exists in the system, but which immediately cuts the connection to the drain 40 if the pressure there is greater than in the remainder of the system. In such a case an air gap is created instead so that it is impossible for fluid to be conducted from the drain 40 into the system.

Preferred Embodiments of pressure equalizing devices used in connection with the Invention

In Fig. 2 is shown a pressure equalizing device which is intended in particular to form the pressure equalizing device 20 in Fig. 1. It has been given, therefore, the designation 20a. Filtrate is supplied to the outer housing 41 of the device via the duct 17a and the inlet 21a and filtrate is withdrawn via the outlet 22a, the duct 23a and the check valve 24a. At the top there is a further connecting nipple 42 with a connecting duct 43 which in the case shown here is closed off by means of a weld 44. This connection can be intended for the withdrawal of samples or for the supply of some reagent or the like. Alternatively it may be used for pressure measurement. Numeral 45 designates a window which is covered by a membrane 46 which preferably is hydrophobic, gas-permeable but liquid-tight. Thanks to this window and the back pressure generated by the check valve 24, the fluid level 47 is adjusted automatically. If the fluid level at the start is located above the window it will drop gently owing to bubbles introduced until it reaches the window. If the fluid level at the start is lower, the gas accumulated is automatically pressed out through the window so that the fluid level rises. As a result the fluid level thus will vary between the upper and lower points of the window or membrane.

The design according to Fig. 3 corresponds in principle to that according to Fig. 2. For this reason the same reference numerals have been used, though with addition of the letter b. The device as a whole thus has been designated 20b and the housing for the same 41b. In the same way the window with associated membrane have been designated 45b and 46b respectively. Thus the filtrate is supplied via the duct 17b and the inlet 21b and is withdrawn via the outlet 22b, the duct 23b and the check valve 24b. The difference in respect of the device according to Fig. 2 consists in that the window 45b with its membrane 46b is arranged horizontally inside the housing 41b and is connected to the outer atmosphere via a duct 48 and an outlet 49.

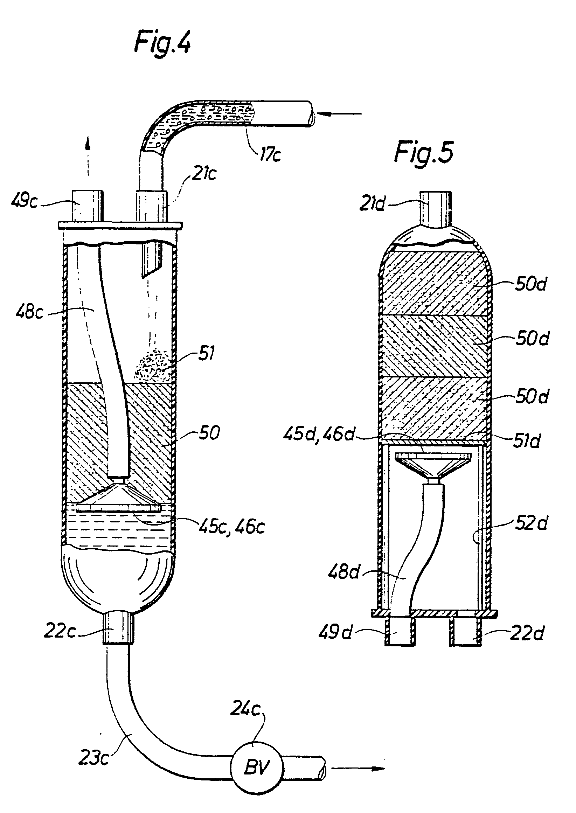

The design according to Fig. 4 corresponds substantially to that according to Fig. 3. For this reason the same reference numerals have been used though replacing the letter b by c. Thus the filtrate is supplied via the duct 17c and the inlet 21c and is withdrawn via the outlet 22c, the duct 23c and the check valve 24c. The gas accumulated is discharged via the window 45c with the membrane 46c via the duct 48c and the outlet 49c. The difference in respect of the design according to Fig. 3 consists mainly in that the device has been provided with a polyurethane foam coated with silicone oil which is designated 50. With the help of this a formation of froth in the filtrate is prevented whilst any froth 51 formed already is broken down.

Although polyurethane foam with silicone oil is a well-proven means for the breaking down of blood froth, it will be clear to those versed in the art that other froth-preventing agents could, of course, also be used.

The design according to Fig. 5 corresponds substantially to that according to Fig. 4. For this reason the same reference numerals have been used, though replacing the letter c by d. Thus the filtrate is supplied via the inlet 21d and is withdrawn via the outlet 22d. The gas accumulated is discharged via the window 45d and the outlet 49d. Any filtrate froth formed is broken down by means of polyurethane froth coated with silicone oil and designated 50d. For practical reasons it has been inserted here in the form of three discs arranged on top of one another. These discs rest on a plate 51d arranged on a supporting leg 52d. As a result the filtrate is made to follow the inner walls of the pressure equalizing device even if the fluid level happens to be lower than the height of the plate 51d. It is an advantage of this design that the duct 48d normally will be immersed in the fluid. In this manner no condensate can be formed on the inside of the duct which is a hazard which may exist for example in the design according to Fig. 4. Such a condensate may cause the inside of the membrane to be blocked for the passage of gas.

Naturally the invention can be varied within the scope of the following claims. Thus, for example, the components included in the system according to the invention can be varied within wide limits in respect of form as well as of function.

## Claims

1. A blood filtering system comprising means (2, 3) for the withdrawal of blood from a patient (1) or some other source, a filter (11) by means of which a filtrate can be withdrawn from the blood, a duct (17) with a pump 19) for sucking the filtrate from the filter (11) to a flow meter (26) as well as suitable means for the return of the remainder of the blood to the source together with any replacement fluid, characterized by a pressure equalizing device (20) arranged in the duct (17) between the pump (19) and the flow meter (26) for equalizing of the pressure in the flow filtrate conducted through the flow meter (26).

2. A system in accordance with claim 1, characterized in that the said pressure equalizing device (20) is situated in close connection to the flow meter (26).

3. A system in accordance with claim 1 or 2, characterized in that the pressure equalizing device (20) is adapted also to capture any gas bubbles occurring in the flow of filtrate.

4. A system in accordance with claim 3, characterized in that the said pump (19) is a peristaltic pump adapted to act from the outside upon the filtrate conducted in a flexible tube.

5. A system in accordance with anyone of the preceding claims, characterized by a measuring device (26c) also for the amount of replacement fluid supplied.

6. A system in accordance with claim 5, characterized by a common measuring device (26a) for the measurement of filtrate as well as of replacement fluid.

7. A system in accordance with claim 6, characterized in that the common measuring device (26a) is adapted to compare the fluid flows measured.

8. A system in accordance with anyone of claims 5-7, characterized by a second pressure equalizing device (36) arranged in a duct (34) for the supply of replacement fluid to the said source (1) and located appropriately downstream the measuring device (26c) for this fluid.

9. A system in accordance with anyone of the preceding claims, characterized in that at least one of the said pressure equalizing devices (20, 36) is provided with a suitable automatic level control.

10. A system in accordance with claim 9, characterized in that the level control is adapted so as to be achieved with the help of a window (45) to the outer atmosphere covered by a membrane (46) which is gas-permeable but liquid-tight.

11. A system in accordance with claim 10, characterized in that the said window (45) is arranged on the outer wall of a housing (41) enclosing the pressure equalizing device with the membrane (46) arranged in vertical position, so that the level normally will vary between the highest and the lowest points of the membrane (46).

12. A system in accordance with claim 10, characterized in that the said window (45b) is arranged inside a housing (41b) enclosing the pressure equalizing device, one side of the membrane being adapted to be in contact with the interior of the housing and its other side with the outer atmosphere.

13. A system in accordance with anyone of the preceding claims, characterized in that at least one of the said pressure equalizing devices (20, 36) is provided with means (5) for the prevention of frothing or for the breaking down of froth already formed.

14. A system in accordance with anyone of the preceding claims, characterized by means (26d) for measuring the temperature of the medium flowing through the said measuring device for a possible adjustment of the measuring result on account of the temperature-sensitive density.

## Patentansprüche

1. Blutfiltriersystem mit einer Einrichtung (2, 3) für die Entnahme von Blut von einem Patienten (1), oder einer anderen Quelle, einem filter (11), mit Hilfe dessen ein Filtrat aus dem Blut abgezogen werden kann, einer Leitung (17) mit einer Pump (19) zum Absaugen des Filtrates von dem Filter (11) zu einem Durchflußgerät (26) und ebenso mit geeigneten Mitteln zum Zurükführen des verbleibenden Blutes zu der Quelle zusammen mit einem Ersatzfluid, gekennzeichnet durch eine Druckausgleichseinrichtung (20), welche in der Leitung (17) zwischen der Pumpe (19) und dem Durchflußmeßgerät (26) angeordnet ist zum Ausgleichen des Druckes in dem Filtratstrom, welcher durch das Flußmeßgerät (26) hindurchgeleitet wird.

2. System nach Anspruch 1, dadurch gekennzeichnet, daß die Druckausgleichseinrichtung (20) in enger Verbindung zu dem Durchflußmeßgerät (26) angeordnet ist.

3. System nach Anspruch 1 oder 2, dadurch gekennzeichnet, daß die Druckausgleichseinrichtung (20) außerdem dafür ausgelegt ist, in dem Filtratstrom auftretende Gasblasen abzufangen.

4. System nach Anspruch 3, dadurch gekennzeichnet, daß die Pumpe (19) eine peristaltische Pumpe ist, welche so ausgelegt ist, daß sie von der Außenseite auf das Filtrat wirkt, welches in einem flexiblen Schlauch geführt ist.

5. System nach einem der vorstehenden Ansprüche, gekennzeichnet durch eine Meßvorrichtung (26c) auch für die Menge des zugeführten Ersatzfluids.

6. System nach Anspruch 5, gekennzeichnet durch eine gemeinsame Meßvorrichtung (26a) sowohl für die Messung von Filtrat als auch für die Messung von Ersatzfluid.

7. System nach Anspruch 6, dadurch gekennzeichnet, daß die gemeinsame Meßvorrichtung (26a) so ausgelegt ist, daß sie die gemessenen Fluidströme miteinander vergleicht.

8. System nach einem der Ansprüche 5 bis 7, gekennzeichnet durch eine zweite Druckausgleichseinrichtung (36), welche in einer Leitung (34) für die Zufuhr von Ersatzfluid zu der Quelle (1) angeordnet ist und zweckmäßigerweise stromabwärts von der Meßvorrichtung (26c) für dieses Fluid liegt.

9. System nach einem der vorstehenden Ansprüche, dadurch gekennzeichnet, daß zumindest eine der Druckausgleichseinrichtungen (20, 36) mit einer geeigneten automatischen Niveaukontrolle bzw.-steuerung versehen ist.

10. System nach Anspruch 9, dadurch gekennzeichnet, daß die Niveaukontrolle so ausgelegt ist, daß sie mit Hilfe eines Fensters (45) zur äußeren Atmosphäre hin bewirkt werden kann, welches durch eine Membran (46) abgedeckt ist, die gasdurchlässig jedoch flüssigkeitsdicht ist.

11. System nach Anspruch 10, dadurch gekennzeichnet, daß das Fenster (45) an der Außenwand eines Gehäuses (41) angeordnet ist, welche die Druckausgleichseinrichtung einschließt, wobei die Membran (46) in einer vertikalen Stellung angeordnet ist, so daß das Niveau normalerweise zwischen den höchsten und niedrigsten Stellen der Membran (46) variiert.

12. System nach Anspruch 10, dadurch gekennzeichnet, daß das Fenster (45b) innerhalb eines Gehäuses (41b) angeordnet ist, welches, die Druckausgleichseinrichtung einschließt, wobei eine Seite der Membran so ausgelegt ist, daß sie mit dem Inneren des Gehäuses in Berührung steht und ihre andere Seite mit der äußeren Atmosphäre in Kontakt ist.

13. System nach einem der vorstehenden Ansprüche, dadurch gekennzeichnet, daß zumindest eine der Druckausgleichseinrichtung (20, 26) mit Mitteln (5) für die Verhinderung von Schaumbildung oder für die Zerstörung von schon gebildeten Schaum versehen ist.

14. System nach einem der vorstehenden Ansprüchen, gekennzeichnet durch eine Einrichtung zum Messen der Temperatur des Mediums, welches durch die Meßvorrichtung hindurchfließt, für eine mögliche Justierung des Meßergebnisses unter Berücksichtigung der temperaturabhängigen Dichte.

## Revendications

1. Système de filtrage du sang comprenant des moyens (2, 3) pour l'extraction du sang d'un patient (1) ou d'une autre source, un filtre (11) au moyen duquel un filtrat peut être extrait du sang, un conduit (17) comportant une pompe (19) pour aspirer le filtrat du filtre (11) vers un débitmètre (26), ainsi que des moyens appropriés pour le retour du reste du sang vers la source en même temps qu'un fluide de remplacement éventuel, caractérisé en ce qu'un dispositif d'égalisation de pression (20) est placé dans le conduit (17) entre la pompe (19) et le débitmètre (26) pour l'égalisation de la pression dans l'écoulement de filtrat conduit à travers le débitmètre (26).

2. Système suivant la revendication 1, caractérisé en ce que ledit dispositif d'égalisation de pression (20) est situé très près du débitmètre (26).

3. Système suivant la revendication 1 ou 2, caractérisé en ce que le dispositif d'égalisation de pression (20) est prévu également pour capturer les éventuelles bulles de gaz apparaissant dans l'écoulement de filtrat.

4. Système suivant la revendication 3, caractérisé en ce que ladite pompe (19) est une pompe péristaltique prévue pour agir de l'extérieur sur le filtrat conduit dans un tube flexible.

5. Système suivant l'une quelconque des revendications précédentes, caractérisé par un dispositif de mesure (26c) également pour la quantité de fluide de remplacement fournie.

6. Système suivant la revendication 5, caractérisé par un dispositif de mesure commun (26a) pour la mesure du filtrat et celle du fluide de remplacement.

7. Système suivant la revendication 6, caractérisé en ce que le dispositif de mesure commun (26a) est prévu pour comparer les débits de fluide mesurés.

8. Système suivant l'une quelconque des revendications 5 à 7, caractérisé en ce qu'un deuxième dispositif d'égalisation de pression (36) est prévu dans un conduit (34) d'amenée de fluide de remplacement à ladite source (1) et il est situé de façon appropriée en aval du dispositif de mesure (26c) pour ce fluide.

9. Système suivant l'une quelconque des revendications précédentes, caractérisé en ce qu'au moins l'un desdits dispositifs d'égalisation de pression (20, 36) comporte un réglage de niveau automatique approprié.

10. Système suivant la revendication 9, caractérisé en ce que le réglage de niveau est prévu pour être effectué à l'aide d'une fenêtre (45) débouchant à l'atmosphère extérieure et couverte d'une membrane (46) qui est perméable aux gaz mais étanche aux liquides.

11. Système suivant la revendication 10, caractérisé en ce que ladite fenêtre (45) est prévue dans la paroi extérieure d'une enveloppe (41) entourant le dispositif d'égalisation de pression, la membrane (46) étant placée en position verticale de sorte que le niveau varie normalement entre le point le plus haut et le point le plus bas de la membrane (46).

12. Système suivant la revendication 10, caractérisé en ce que ladite fenêtre (45b) est agencée à l'intérieur d'une enveloppe (41b) entourant le dispositif d'égalisation de pression, une face de la membrane étant disposée de manière à être en contact avec l'intérieur de l'enveloppe et son autre face avec l'atmosphère extérieure.

13. Système suivant l'une quelconque des revendications précédentes, caractérisé en ce qu'au moins l'un desdits dispositifs d'égalisation de pression (20, 36) comporte des moyens (5) pour la prévention du moussage ou pour briser la mousse déjà formée.

14. Système suivant l'une quelconque des revendications précédentes, caractérisé per des moyens (26d) de mesure de la température du fluide circulant dans ledit dispositif de mesure, pour une correction possible du résultat de mesure afin de tenir compte de la sensibilité de la densité à la température.

*Fig.1*

EP 0 165 519 B1

# Fig.2

# Fig.3

Fig.4

Fig.5